# EUROPEAN PATENT SPECIFICATION

(11) **EP 3 492 050 B1**
(45) Date of publication and mention of the grant of the patent: **15.04.2020**
(21) Application number: 18207965.7
(22) Date of filing: 23.11.2018
(51) Int. Cl.: A61F 5/01

(54) **ARTICULATED ROD OF AN ORTHOPAEDIC DEVICE**
GELENKSTANGE EINER ORTHOPÄDISCHEN VORRICHTUNG
TIGE ARTICULÉE D'UN DISPOSITIF ORTHOPÉDIQUE

(30) Priority: 29.11.2017 IT 201700136919
(43) Date of publication of application: 05.06.2019
(73) Proprietor: Orthoservice AG, 6830 Chiasso (CH)
(72) Inventor: TURCONI, Francesco, 20900 MONZA (MB) (IT); ALBERIO, Daniele, 23898 IMBERSAGO (LC) (IT)
(74) Representative: Branca, Emanuela

(56) References cited:
- DE-U1-202011 102 331
- US-A- 4 732 143
- US-A1- 2006 287 624

## Description

The present invention refers to an articulated rod of an orthopaedic device.

In the state of the art there are articulated rods of orthopaedic devices with polycentric joints that can be adjusted in flexion-extension comprising inserts for constraining at least a flexion or extension rotation within a rotation width. The insert is associated with the polycentric joint through the use of screws that make the mounting and replacement step of the inserts on the orthopaedic device disadvantageously complex.

The presence of threaded holes and screws makes it difficult for the orthopaedic personnel needing to arrange suitable tools to apply the inserts.

Other locking elements of the inserts disadvantageously increase the size of the articulated rod which should instead be small and as compact as possible since it is an element of the orthopaedic device.

An articulated rod in accordance with the preamble of claim 1 is disclosed in DE 20 2011 102331 U1.

The object of the present invention is to provide an articulated rod in which the inserts are stably maintained in position during the use of an orthopaedic device, the inserts can be easily replaced for adjusting an extension-flexion rotation width and the articulated rod is compact, stable and small.

According to the present invention said object is achieved by providing an articulated rod as disclosed in claim 1.

Further characteristics are highlighted in the dependent claims.

The characteristics and advantages of an articulated rod with a polycentric joint that can be adjusted in extension-flexion according to the present invention will be more apparent from the following description, which is to be understood as exemplifying and not limiting, with reference to the schematic attached drawings, wherein:
Figure 1 shows a partially exploded perspective view of an articulated rod of an orthopaedic device with a polycentric joint;
Figure 2 shows an exploded front perspective view of the articulated rod comprising a guide base, two half covers and the locking bar;
Figure 3 shows an exploded rear perspective view of the articulated rod;
Figure 4 shows a perspective view from below of the locking bar;
Figure 5 shows a perspective view from above of a guide base;
Figure 6 shows a perspective view from below of the guide base;
Figure 7 shows a perspective view from below of two half covers;
Figure 8 shows a plan view from below of an open position of the two half covers mounted with the guide base and with the locking bar;
Figure 9 shows a plan view from below of a closed position of the two half covers mounted with the guide base and with the locking bar.

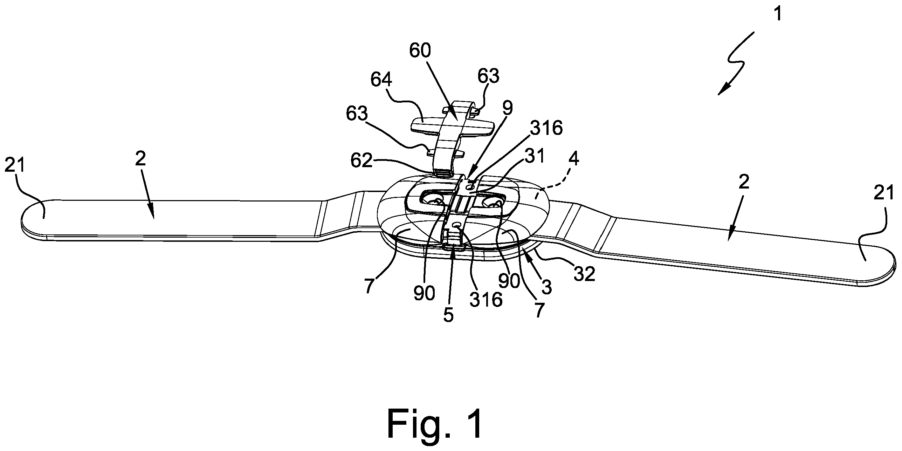

With particular reference to Figures 1-3, an articulated rod 1 of an orthopaedic device is shown comprising a pair of arms 2, two cover plates 3 and a polycentric joint 4 that is mounted with the two cover plates 3 and articulates the pair of arms 2.

The two cover plates 3 are an outer cover plate 31 and an inner cover plate 32, where the terms inner and outer refer to mounting directions with respect to the orthopaedic device.

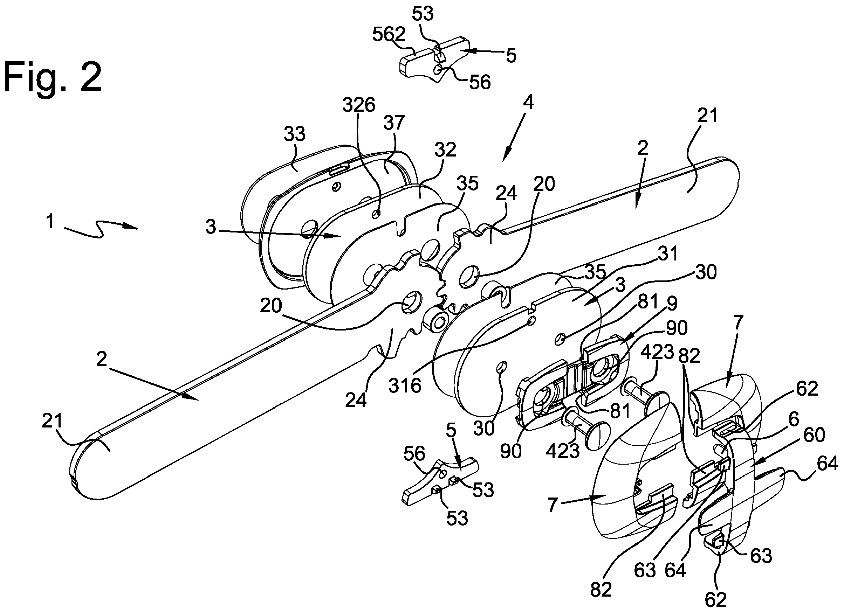

As shown in Figure 2, the two cover plates 31, 32 mount a gasket 35 for promoting the movement of the polycentric joint 4. The inner cover plate 32 envisages the mounting of an inner cover 37 comprising edges that match with the pair of half covers 7. Towards the inside the inner cover 37 mounts a piece of Velcro 33.

In general the polycentric joint 4 allows a mutual rotation of the pair of arms 2. The rotation comprises two rotation directions which are a direction of flexion rotation and a direction of extension rotation.

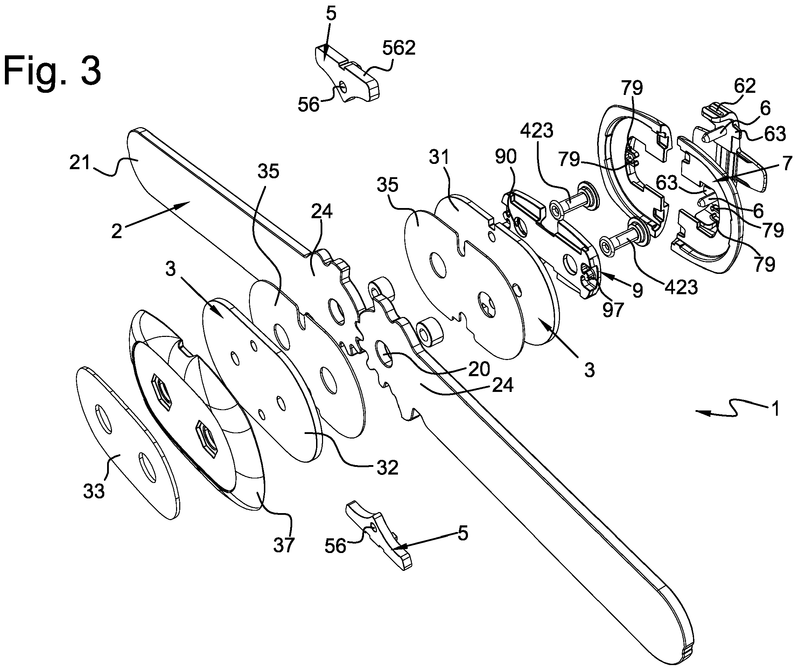

In the embodiment shown in particular in Figure 3 each arm 2 bears a first end 21 that can be associated with the orthopaedic device and a toothed end 24 opposite the first end 21.

The toothed ends 24 of the pair of arms 2 are meshed together for performing the rotation on a geometric plane of rotation coplanar thereto. Each toothed end 24 bears a central hole 20 in the axial position with respect to the extension of the toothed profile.

The two cover plates 3 are coupled to each other to form a shell and house the two toothed ends 24 and two rotation pins 423. The two rotation pins 423 are arranged orthogonally with respect to the geometric plane of rotation. The two pins are respectively engaged in central holes 20 of the arms 2 and in the through holes 30 of the two cover plates 3.

As shown in Figure 3, the articulated rod 1 further comprises two removable inserts 5. Each insert 5 is selectively engageable in a volume comprised between the two plates 3 and the arms 2.

The flexion-extension rotation width of the pair of arms 2 is not constrained by the insert 5 when the insert 5 is not engaged in the polycentric joint 4.

Figure 3 shows an example of the insert 5 that has a wedge shape for the engagement of the two arms 2. Projections 53 of the insert 5 allow the preliminary centring of the insert 5 with respect to the plate 31. Inserts 5 of different shapes or sizes may envisage allowing a plurality of flexion and extension rotation widths of the two arms 2. It follows that the flexion rotation width may also be different from the extension rotation width.

The articulated rod 1 also comprises two stop pins 6, i.e. one stop pin 6 for every insert 5. Each stop pin 6 is engageable in the respective insert 5 and with at least one of the cover plates 3.

Even more advantageously, as in the example described in Figures 1-9, it is possible to envisage each stop pin 6 engaging the insert 5 with both of the cover plates 3 so as to make the engagement even more secure.

The stop pin 6 has a longitudinal dimension that extends along an orthogonal direction to the geometric plane of rotation.

To place the stop pin 6 in position, the insert 5 bears a through hole 56, the outer cover plate 31 bears a through hole 316, the inner cover plate 32 bears a through hole 326, where such through holes 56, 316 and 326 are aligned with each other so as to be engaged by the stop pin 6 in position. The through holes 56, 316 and 326 are not threaded and the stop pin 6 is not a screw, advantageously simplifying the block.

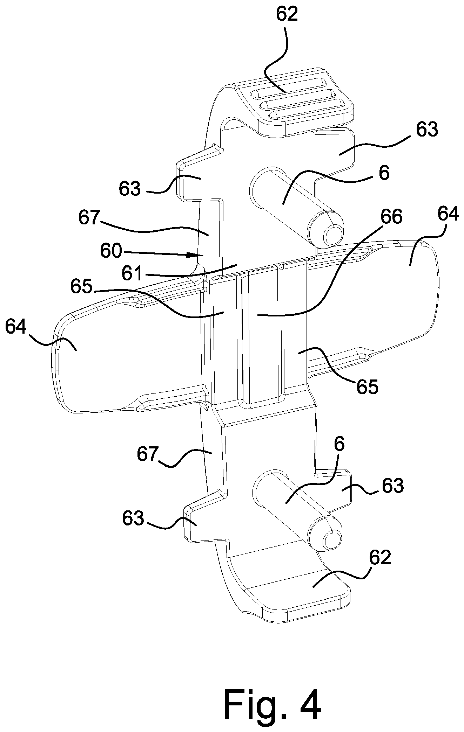

Even more advantageously, the two stop pins 6 can simultaneously engage the two inserts 5 with at least one of the two plates 3, and it is therefore envisaged that the two stop pins 6 are a single piece with a locking bar 60, as shown in Figure 4, or that the two stop pins 6 are mounted solidly with the locking bar 60, an operation that would not be possible to actuate with locking screws of the state of the art as they need to be screwed separately. Mounting the two stop pins 6 on a single locking bar 60 allows the inserts 5 locking operation to be quicker, more secure and easier.

As shown in particular in Figure 4, the locking bar 60 comprises a base 61 that rests on a geometric plane parallel to the geometric plane of rotation.

From the base 61 the two stop pins 6 are parallel to each other and project orthogonally to the geometric plane of the base 61.

The locking bar 60 comprises at the ends thereof two walls 62 substantially orthogonal to the base 61 that extend for a height sufficient to at least partially cover a lateral thickness 562 of the insert 5 so that each wall 62 is a containing element and provides greater security for maintaining the insert 5 even more firmly in position in the polycentric joint 4.

Each wall 62 has a prehensile portion for making it advantageously easy to position and remove the locking bar 60 from its own seat.

The locking bar 60 further comprises two pairs of fins 63. Each fin 63 is an extension of the base 61 and lies on a geometric plane parallel to the geometric plane of the base 61. In the embodiment shown in particular in Figure 4, the geometric plane on which the fins 63 lie is the same geometric plane on which the base 61 of the locking bar 60 lies, but these two geometric planes may also be at different heights.

Each pair of fins 63 is advantageously arranged in proximity to the respective stop pin 6 so as to reduce the clearances of the locking bar 60 and allow the positioning of the stop pins 6 more securely in the holes 56, 316, 326.

According to the embodiment shown in the figures, the locking bar 60 also comprises a pair of covers 64 that cover the two rotation pins 423.

The locking bar 60 comprises interconnection elements that are grooves 65 and a step 66, which are arranged in the central part of the locking bar 60.

The locking bar 60 has side walls 67.

As shown in Figures 2, 3, 5, 6, the articulated rod 1 comprises a guide base 9 that is integral with the outer cover plate 31.

The guide base 9 comprises two through holes 90 at the central holes 20 and the through holes 30. The two through holes 90 are engaged by the two rotation pins 423 that make the guide base 9 integral with the outer cover plate 31, as shown in Figures 1-3.

According to a further embodiment, not shown in the figures, the guide base 9 could be made as a single piece with the outer cover plate 31, or completely absent.

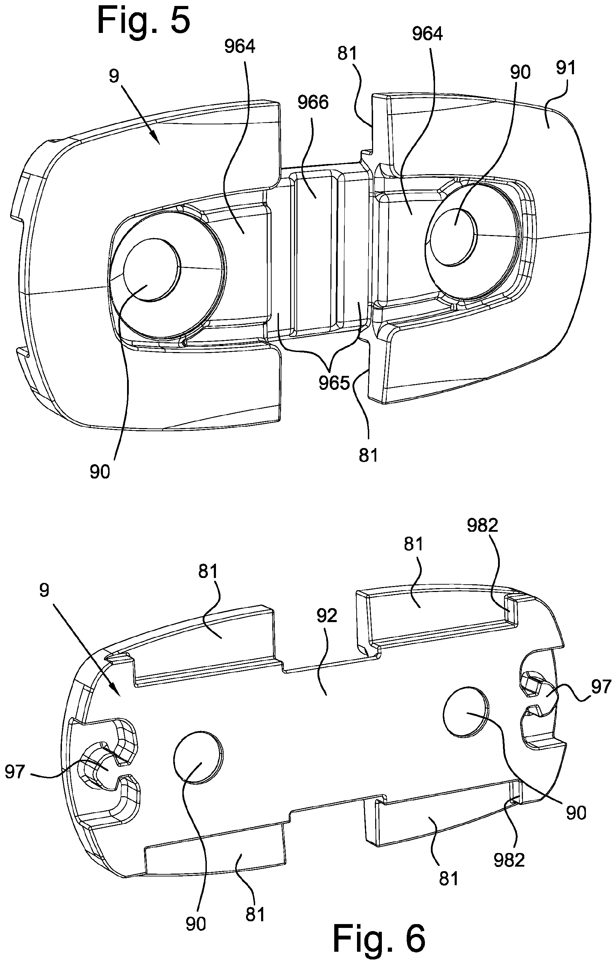

The guide base 9 comprises an outer face 91, shown in Figure 5, and an inner face 92, shown in Figure 6. The outer face 91 comes into contact with the locking bar 60. The inner face 92 is in contact with the outer cover plate 31.

The guide base 9 comprises interconnection elements which are steps 965 and a groove 966. In particular, the steps 965 and the grooves 966 are provided on the outer face 91.

A coupling between the grooves 65 of the locking bar 60 and the steps 965 of the guide base 9 and a related coupling between the step 66 of the locking bar 60 and the grooves 966 of the guide base 9 allow the locking bar 60 to be advantageously positioned more securely and without any clearance and therefore the stop pins 6 to be positioned more securely and the inserts 5 to be kept more stably positioned without any clearances.

Alternatively it is possible to envisage the steps 965 and the groove 966 being in a single piece with the outer cover plate 31.

The outer face 91 of the guide base 9 bears a pair of grooves 964 adapted to house the pair of covers 64 of the locking bar 60 so that the pair of covers 64 advantageously covers the through holes 90 of the guide base 9. Even more advantageously the thickness of the covers 64 corresponds to the height of the grooves 964 so that the outer surface of the articulated rod 1 is advantageously smooth and free from projections or grooves that could get caught in the use of the orthopaedic device.

As shown in particular in Figures 2-3, 5-9, the articulated rod 1 comprises a pair of half covers 7 and guide means 81, 82 coupled to each other for slidably associating the pair of half covers 7.

As shown in particular in Figures 5 and 6, the guide base 9 bears a first element 81 of the guide means and each of the half covers 7 bears a second element 82 of the guide means. In this way, the two half covers 7 are slidably associated with the guide base 9.

With particular reference to Figure 6, the first element 81 of the guide means comprises two pairs of grooves 81 of the guide base 9. The inner face 92 bears a first pair of grooves 81 arranged at one end of the guide base 9 for guiding a first half cover 7 and a second pair of grooves 81 arranged at an opposite end of the guide base 9 for guiding a second half cover 7.

As shown in particular in Figure 6, the guide base 9 also comprises two pairs of stroke end stops 982, i.e. a pair of stroke end stops 982 for each end of the guide base 9, so that each of the half covers 7 can slide on the first element 81 of the guide means from a closed position of the half cover 7 to an open position of the half cover 7, being it constrained to the guide base 9 by the stroke end stops 982.

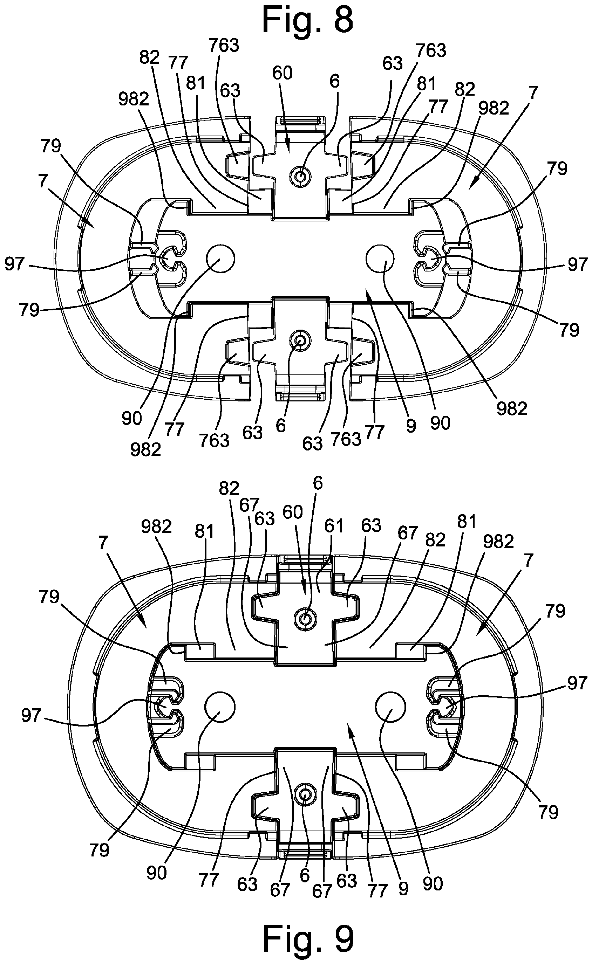

As shown in particular in Figure 8, the open position of the half cover 7 envisages that the half cover 7 can slide open until it abuts with the pair of stroke end stops 982 of the guide base 9.

As shown in particular in Figure 9, the closed position of the half cover 7 envisages that the half cover 7 can slide closed until it abuts with the respective side wall 67 of the locking bar 60.

As shown in particular in Figure 6, the guide base 9 bears a pair of interlocking elements 97 for the respective engagement elements 79 of the half covers 7. Each end of the guide base 9 bears the interlocking element 97. Each half cover 7 bears the engagement element 79 adapted to fit with the respective interlocking element 97 of the guide base 9 so as to advantageously secure the half cover 7 with the guide base 9.

Alternatively it is possible to envisage that each end of the guide base 9 can bear a plurality of interlocking elements 97 and that a respective plurality of engagement elements 79 of the half covers 7 is provided.

As shown in particular in Figures 6, 8 and 9, the interlocking element 97 is a mushroom-shaped extension.

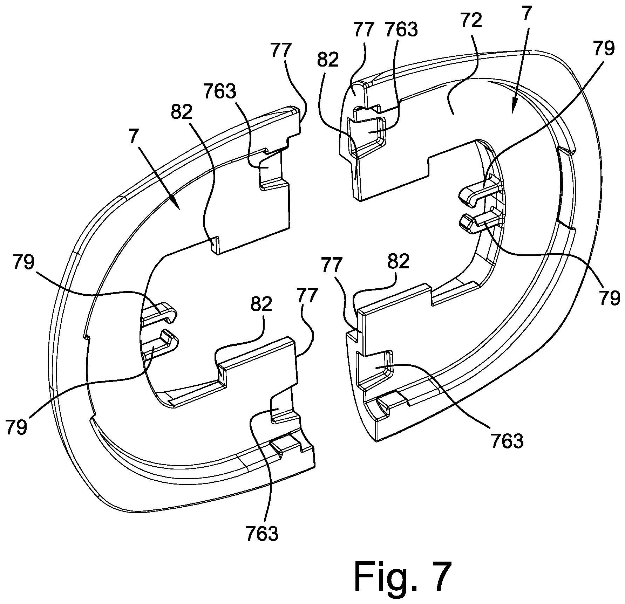

As shown in particular in Figures 7, 8 and 9, the engagement element 79 of the half cover 7 comprises two elastic extensions that engage the mushroom shape of the respective interlocking element 97 of the guide base 9.

As shown in particular in Figures 2-3 and 7-9, each half cover 7 of the pair of half covers 7 bears the second element 82 of the guide means.

The second element 82 of the guide means is an extension of the half cover 7 arranged on a geometric plane at a different height with respect to the geometric plane on which the first element 81 of the guide means is arranged, so that the first element 81 acts as a guide for the sliding of the second element 82 of the guide means.

The half covers 7 pass from a closed position interfering with the stop pins 6 to an open position free from interference with the stop pins 6.

The closed position of the half cover 7 envisages that at least one element 763 of the half cover 7 enters into interference with at least one portion 63 that is integral with the at least one stop pin 6 locking the at least one stop pin 6 in engagement with the polycentric joint 4.

The open position of the half covers 7 envisages that no element of the half cover 7 enters into interference with any portion of the at least one stop pin 6, allowing the removal of the at least one stop pin 6 from the polycentric joint 4.

As shown in particular in Figures 7-9, each half cover 7 has an inner face 72 that bears two grooves 763 at the respective pairs of fins 63 of the locking bar 60, so that once the two half covers 7 are in the closed position, as shown in Figure 9, the fins 63 are locked by the grooves 763 of the half covers 7, as the fins 63 are covered by the grooves 763 preventing the removal, which may even be accidental, of the stop pin 6 from the polycentric joint 4. When the half covers 7 are in the closed position, the coupling between the grooves 763 and the fins 63 advantageously allows the removal of the stop pins 6 from the polycentric joint 4 to be prevented.

Even more advantageously it is envisaged that the grooves 763 are arranged at an abutment portion 77 of the two half covers 7, so that when the half covers 7 are in the closed position the abutment portion 77 abuts against side walls 67 of the locking bar 60 so as not to leave any gaps and the outer surface of the articulated rod is smooth, so as not to get caught during use of the orthopaedic device.

In relation to the adjustment of the articulated rod 1 by the orthopaedic personnel based on the specific needs of the patient, with the half covers in the open position, the inserts 5 are positioned in the polycentric joint 4. Subsequently, the locking bar 60 inserts the stop pins 6 into the through holes 316, 56 and 326 locking the inserts 5 in the polycentric joint 4. As shown in Figures 8 and 9, the two half covers 7 are made to pass from the open position to the closed position ensuring that the engagement and interlocking elements 79 and 97 block the mutual movement of the two half covers 7 firmly and securely maintaining the stop pins 6 and the respective inserts 5 in the polycentric joint 4.

Advantageously the articulated rod 1, according to the invention, has inserts 5 that are easily replaceable to adjust the flexion-extension rotation width, hence being easily and quickly mountable, secure, compact and small.

Alternatively the outer face 91 bears the first element 81 of the guide means. The second element 82 of the guide means has a complementary shape to the first element 81, so as to allow the sliding of the half covers 7 with the guide base 9.

Alternatively the outer cover plate 31 bears the first element 81 of the guide means. In this alternative the half covers 7 are slidably associated with the outer cover 31.

Even more alternatively it is possible to envisage that only one of the two half covers 7 is slidably associated with the guide base 9 and the other half cover 7 remains integral with the guide base 9.

Alternatively it is envisaged that the half covers 7 comprise an interlocking element and a respective engagement element so that the two half covers 7 can be secured directly to each other in the closed position, e.g. by interlocking, rather than being secured to the guide base 9. In this case it is envisaged that the half covers 7 cover at least one portion of the stop pins 6 so that the stop pins 6 cannot exit from the through holes 56, 316, 326, the at least one portion of the stop pins 6 being in interference by abutment with at least one portion of the half covers 7. For example, it is predictable that the half covers 7 in the closed position cover the locking bar 60 fully or partly to prevent it exiting its seat.

Alternatively it is sufficient that only one half cover 7 of the pair of half covers 7 is adapted to pass from the closed position to the open position. In this alternative the closed position envisages that at least one element 763 of one of the two half covers 7 enters into interference with at least one portion 63 of the at least one stop pin 6 blocking the at least one stop pin 6 or both of the stop pins 6.

The articulated rod 1 according to the invention can be applied indifferently to orthopaedic devices for the upper or lower limbs.

The articulated rod thus conceived is susceptible to many modifications and variations, all falling within the invention; furthermore, all the details are replaceable by technically equivalent elements. In practice, the materials used, as well as the dimensions, can be any according to the technical requirements.

The scope of the invention, however, is solely defined by the appended claims.

## Claims

1. Articulated rod (1) of an orthopaedic device comprising a pair of arms (2), two cover plates (3), a polycentric joint (4) articulating said pair of arms (2) in mutual rotation, where said rotation comprises a flexion rotation direction and an extension rotation direction, at least one removable insert (5), selectively engageable so as to block said flexion and/or extension rotation within a predetermined angular width, at least one stop pin (6) adapted to be in position axially engaging at least one through hole (56) of said at least one insert (5) and at least one through hole (316, 326) of at least one of the two cover plates (3),
**characterised in that** said rod further comprises
a pair of half covers (7) and guide means (81, 82) coupled to each other in order to slidably associate at least one half cover (7) of said pair of half covers (7), where said guide means (81, 82) comprise a first element (81) integral with said at least one cover plate (3) and a second element (82) integral with said at least one half cover (7), said pair of half covers (7) being adapted to pass from an open position to a closed position, where said closed position provides for at least one element of said at least one half cover (7) to enter into interference with at least one portion of said at least one stop pin (6) holding in position said at least one stop pin (6) with respect to the translation along its own axis.

2. Articulated rod (1) according to claim 1, **characterised in that** it further comprises a guide base (9) which is integral with said at least one cover plate (3), said guide base (9) bears said first element (81) of the guide means which has a complementary shape with respect to said second element (82) of the guide means so as to allow the sliding of said at least one half cover (7) with said guide base (9).

3. Articulated rod (1) according to any one of claims 1 or 2, **characterised in that** it further comprises a locking bar (60) which bears two mutually parallel stop pins (6), adapted to engage two inserts (5) simultaneously.

4. Articulated rod (1) according to claim 3, **characterised in that** said locking bar (60) comprises, at its ends, two walls (62) extended for a height sufficient to at least partially cover a lateral thickness (562) of said at least one insert (5).

5. Articulated rod (1) according to any one of claims 3 or 4, **characterised in that** said at least one element of said at least one half cover (7) is at least one groove (763) of said at least one half cover (7) and that said at least one portion of said at least one stop pin (6) is at least one fin (63) of said locking bar (60), where the closed position of said at least one half cover (7) provides for said at least one fin (63) to be covered by said at least one groove (763).

6. Articulated rod (1) according to any one of claims 3-5, **characterised in that** said guide base (9) comprises interconnection elements (965, 966) adapted to be coupled with respective interconnection elements (65, 66) of the locking bar (60) when said at least one stop pin (6) is positioned.

7. Articulated rod (1) according to any one of claims 1-6, **characterised in that** the closed position of said pair of half covers (7) provides for each half cover (7) to be secured to the other half cover (7) of said pair of half covers (7).

8. Articulated rod (1) according to any one of claims 2-6, **characterised in that** the closed position of said pair of half covers (7) provides for each half cover (7) of said pair of half covers (7) to be secured with said guide base (9).

9. Articulated rod (1) according to claim 8, **characterised in that** said guide base (9) bears at least one interlocking element (97) for each half cover (7) of said pair of half covers (7) and that each half cover (7) bears at least one engagement element (79), where the closed position of the pair of half covers (7) provides for said at least one engagement element (79) to be adapted to engage with said interlocking element (97) of the guide base (9).

10. Articulated rod according to claim 9, **characterised in that** the interlocking element (97) of the guide base (9) has a mushroom shape and that the engagement element (79) of the half cover (7) comprises two elastic extensions, where the closed position of the pair of half covers (7) provides for said two elastic extensions to engage the mushroom shape of the respective interlocking element (97) of the guide base (9).

## Patentansprüche

1. Gelenkstange (1) einer orthopädischen Vorrichtung, umfassend ein Paar von Armen (2), zwei Abdeckplatten (3), ein polyzentrisches Gelenk (4), das das Paar von Armen (2) in gegenseitiger Drehung gelenkig verbindet, wobei die Drehung eine Beugungs-Drehrichtung und eine Streckungs-Drehrichtung umfasst, mindestens einen abnehmbaren Einsatz (5), der selektiv in Eingriff bringbar ist, um die Beugungs- und/oder Streckungs-Drehung innerhalb einer vorbestimmten Winkelbreite zu sperren, mindestens einen Anschlagstift (6), der so angepasst ist, dass er durch axiales Eingreifen in mindestens ein Durchgangsloch (56) des mindestens einen Einsatzes (5) und in mindestens ein Durchgangsloch (316, 326) von mindestens einer der beiden Abdeckplatten (3) in Position gehalten wird, **dadurch gekennzeichnet, dass** die Stange ferner ein Paar Halbdeckel (7) und Führungsmittel (81, 82) umfasst, die miteinander gekoppelt sind, um mindestens einen Halbdeckel (7) des Paars von Halbdeckeln (7) gleitend zu verbinden, wobei die Führungsmittel (81, 82) ein erstes Element (81), das mit der mindestens einen Abdeckplatte (3) einstückig ist, und ein zweites Element (82), das mit dem mindestens einen Halbdeckel (7) einstückig ist, umfassen, wobei das Paar von Halbdeckeln (7) so angepasst ist, dass es von einer offenen Position in eine geschlossene Position übergeht, wobei die geschlossene Position dafür sorgt, dass mindestens ein Element des mindestens einen Halbdeckels (7) mit mindestens einem Teil des mindestens einen Anschlagstiftes (6) in Wechselwirkung tritt, wobei der mindestens eine Anschlagstift (6) in Bezug auf die Translation entlang seiner eigenen Achse in Position gehalten wird.

2. Gelenkstange (1) gemäß Anspruch 1, **dadurch gekennzeichnet, dass** sie ferner eine Führungsbasis (9) umfasst, die mit der mindestens einen Abdeckplatte (3) einstückig ist, wobei die Führungsbasis (9) das erste Element (81) des Führungsmittels trägt, das eine komplementäre Form in Bezug auf das zweite Element (82) des Führungsmittels aufweist, um das Gleiten des mindestens einen Halbdeckels (7) mit der Führungsbasis (9) zu ermöglichen.

3. Gelenkstange (1) gemäß einem der Ansprüche 1 oder 2, **dadurch gekennzeichnet, dass** sie ferner einen Verriegelungsstab (60) umfasst, der zwei zueinander parallele Anschlagstifte (6) trägt, die dazu angepasst sind, mit zwei Einsätzen (5) gleichzeitig in Eingriff zu kommen.

4. Gelenkstange (1) gemäß Anspruch 3, **dadurch gekennzeichnet, dass** der Verriegelungsstab (60) an seinen Enden zwei Wandungen (62) umfasst, die sich bis zu einer Höhe erstrecken, die ausreichend ist, um zumindest teilweise eine seitliche Stärke (562) des mindestens einen Einsatzes (5) zu überdecken.

5. Gelenkstange (1) gemäß einem der Ansprüche 3 oder 4, **dadurch gekennzeichnet, dass** das mindestens eine Element des mindestens einen Halbdeckels (7) mindestens eine Nut (763) des mindestens einen Halbdeckels (7) ist und dass der mindestens eine Teil des mindestens einen Anschlagstiftes (6) mindestens eine Rippe (63) des Verriegelungsstabes (60) ist, wobei die geschlossene Position des mindestens einen Halbdeckels (7) dafür sorgt, dass die mindestens eine Rippe (63) von der mindestens einen Nut (763) überdeckt wird.

6. Gelenkstange (1) gemäß einem der Ansprüche 3-5, **dadurch gekennzeichnet, dass** die Führungsbasis (9) Verbindungselemente (965, 966) umfasst, die dazu angepasst sind, mit entsprechenden Verbindungselementen (65, 66) des Verriegelungsstabes (60) gekoppelt zu werden, wenn der mindestens eine Anschlagstift (6) positioniert ist.

7. Gelenkstange (1) gemäß einem der Ansprüche 1-6, **dadurch gekennzeichnet, dass** die geschlossene Position des Paars von Halbdeckeln (7) vorsieht, dass jeder Halbdeckel (7) an dem anderen Halbdeckel (7) des Paars von Halbdeckeln (7) gesichert wird.

8. Gelenkstange (1) gemäß einem der Ansprüche 2-6, **dadurch gekennzeichnet, dass** die geschlossene Position des Paars von Halbdeckeln (7) vorsieht, dass jeder Halbdeckel (7) des Paars von Halbdeckeln (7) an der Führungsbasis (9) gesichert wird.

9. Gelenkstange (1) gemäß Anspruch 8, **dadurch gekennzeichnet, dass** die Führungsbasis (9) mindestens ein Verriegelungselement (97) für jeden Halbdeckel (7) des Paars von Halbdeckeln (7) trägt und dass jeder Halbdeckel (7) mindestens ein Eingriffselement (79) trägt, wobei die geschlossene Position des Paars von Halbdeckeln (7) dafür sorgt, dass das mindestens eine Eingriffselement (79) so angepasst wird, dass es mit dem Verriegelungselement (97) der Führungsbasis (9) in Eingriff kommt.

10. Gelenkstange gemäß Anspruch 9, **dadurch gekennzeichnet, dass** das Verriegelungselement (97) der Führungsbasis (9) eine Pilzform aufweist und dass das Eingriffselement (79) des Halbdeckels (7) zwei federnde Fortsätze umfasst, wobei die geschlossene Position des Paars von Halbdeckeln (7) dafür sorgt, dass die beiden federnden Fortsätze in die Pilzform des jeweiligen Verriegelungselements (97) der Führungsbasis (9) eingreifen.

## Revendications

1. Tige articulée (1) d'un dispositif orthopédique comprenant une paire de bras (2), deux plaques de recouvrement (3), une articulation polycentrique (4) articulant ladite paire de bras (2) en rotation mutuelle, où ladite rotation comprend une direction de rotation de flexion et une direction de rotation d'extension, au moins un insert amovible (5), apte à être mis en prise de manière sélective de façon à bloquer ladite rotation de flexion et/ou d'extension à l'intérieur d'une largeur angulaire prédéterminée, au moins une goupille d'arrêt (6) conçue pour être, en position, en prise de manière axiale au moins un trou traversant (56) dudit au moins un insert (5) et au moins un trou traversant (316, 326) d'au moins l'une des deux plaques de recouvrement (3), **caractérisé en ce que** ladite tige comprend en outre une paire de demi-couvercles (7) et des moyens de guidage (81, 82) couplés l'un à l'autre afin d'associer de manière coulissante au moins un demi-couvercle (7) de ladite paire de demi-couvercles (7), où lesdits moyens de guidage (81, 82) comprennent un premier élément (81) solidaire de ladite au moins une plaque de recouvrement (3) et un second élément (82) solidaire dudit au moins une demi-couvercle (7), ladite paire de demi-couvercles (7) étant conçue pour passer d'une position ouverte à une position fermée, où ladite position fermée permet à au moins un élément dudit au moins un demi-couvercle (7) d'entrer en interférence avec au moins une partie de ladite au moins une goupille d'arrêt (6) en maintenant en position ladite au moins une goupille d'arrêt (6) par rapport à la translation le long de son propre axe.

2. Tige articulée (1) selon la revendication 1, **caractérisée en ce qu'**elle comprend en outre une base de guidage (9) qui est solidaire de ladite au moins une plaque de recouvrement (3), ladite base de guidage (9) porte ledit premier élément (81) des moyens de guidage qui possède une forme complémentaire par rapport audit second élément (82) des moyens de guidage de façon à permettre le coulissement dudit au moins un demi-couvercle (7) avec ladite base de guidage (9).

3. Tige articulée (1) selon l'une quelconque des revendications 1 ou 2, **caractérisée en ce qu'**elle comprend en outre une barre de verrouillage (60) qui porte deux goupilles d'arrêt mutuellement parallèles (6), conçues pour venir en prise avec deux inserts (5) simultanément.

4. Tige articulée (1) selon la revendication 3, **caractérisée en ce que** ladite barre de verrouillage (60) comprend, à ses extrémités, deux parois (62) étendues sur une hauteur suffisante pour recouvrir au moins partiellement une épaisseur latérale (562) dudit au moins un insert (5).

5. Tige articulée (1) selon l'une quelconque des revendications 3 ou 4, **caractérisée en ce que** ledit au moins un élément dudit au moins un demi-couvercle (7) est au moins une rainure (763) dudit au moins un demi-couvercle (7) et **en ce que** ladite au moins une partie de ladite au moins une goupille d'arrêt (6) est au moins une ailette (63) de ladite barre de verrouillage (60), où la position fermée dudit au moins un demi-couvercle (7) permet à ladite au moins une ailette (63) d'être recouverte par ladite au moins une rainure (763).

6. Tige articulée (1) selon l'une quelconque des revendications 3 à 5, **caractérisée en ce que** ladite base de guidage (9) comprend des éléments d'interconnexion (965, 966) conçus pour être couplés avec des éléments d'interconnexion respectifs (65, 66) de la barre de verrouillage (60) lorsque ladite au moins une goupille d'arrêt (6) est positionnée.

7. Tige articulée (1) selon l'une quelconque des revendications 1 à 6, **caractérisée en ce que** la position fermée de ladite paire de demi-couvercles (7) permet à chaque demi-couvercle (7) d'être arrimé à l'autre demi-couvercle (7) de ladite paire de demi-couvercles (7).

8. Tige articulée (1) selon l'une quelconque des revendications 2 à 6, **caractérisée en ce que** la position fermée de ladite paire de demi-couvercles (7) permet à chaque demi-couvercle (7) de ladite paire de demi-couvercles (7) d'être arrimé avec ladite base de guidage (9).

9. Tige articulée (1) selon la revendication 8, **caractérisée en ce que** ladite base de guidage (9) porte au moins un élément d'interverrouillage (97) pour chaque demi-couvercle (7) de ladite paire de demi-couvercles (7) et **en ce que** chaque demi-couvercle (7) porte au moins un élément de mise en prise (79), où la position fermée de la paire de demi-couvercles (7) permet audit au moins un élément de mise en prise (79) d'être conçu pour venir en prise avec ledit élément d'interverrouillage (97) de la base de guidage (9).

10. Tige articulée selon la revendication 9, **caractérisée en ce que** l'élément d'interverrouillage (97) de la base de guidage (9) possède une forme de champignon et **en ce que** l'élément de mise en prise (79) du demi-couvercle (7) comprend deux extensions élastiques, où la position fermée de la paire de demi-couvercles (7) permet auxdites deux extensions élastiques de venir en prise avec la forme de champignon de l'élément d'interverrouillage respectif (97) de la base de guidage (9).
